# EUROPEAN PATENT SPECIFICATION

(11) **EP 2 240 172 B1**
(45) Date of publication and mention of the grant of the patent: **19.03.2014**
(21) Application number: 08864891.0
(22) Date of filing: 18.12.2008
(51) Int. Cl.: A61K 31/196, A61K 31/506, A61K 45/06, A61P 35/02

(54) **COMBINATION OF NILOTINIB AND CHLORAMBUCIL FOR THE TREATMENT OF CHRONIC LYMPHOCYTIC LEUKEMIA**
KOMBINATION VON NILOTINIB UND CHLORAMBUCIL FÜR DIE BEHANDLUNG VON CHRONISCHER LYMPHOZYTISCHER LEUKÄMIE
COMBINAISON DE NILOTINIB ET CHLORAMBUCILE POUR LE TRAITEMENT DE LA LEUCÉMIE LYMPHATIQUE CHRONIQUE

(30) Priority: 21.12.2007 US 15704
(43) Date of publication of application: 20.10.2010
(73) Proprietor: Novartis AG, 4056 Basel (CH)
(72) Inventor: ALOYZ, Raquel Silvia, Montreal Québec H2W 1A2 (CA); PANASCI, Lawrence Carl, Dollard Des Ormeaux Québec H9B 2Z4 (CA)
(74) Representative: Roth, Peter Richard
(86) International application number: PCT/US2008/087336
(87) International publication number: WO 2009/082662

(56) References cited:
- US-A1- 2006 122 186
- ALOYZ R ET AL: "STI571 SENSITIZED CLL LYMPHOCYTES TO CHLORAMBUCIL" 11 July 2003 (2003-07-11), PROCEEDINGS OF THE ANNUAL MEETING OF THE AMERICAN ASSOCIATION FOR CANCER RESEARCH, NEW YORK, NY, PAGE(S) 1107 , XP001179572 ISSN: 0197-016X the whole document
- ALVARADO YESID ET AL: "Emerging therapeutic options for Philadelphia-positive acute lymphocytic leukemia" 1 March 2007 (2007-03-01), EXPERT OPINION ON EMERGING DRUGS, ASHLEY PUBLICATIONS, GB, PAGE(S) 165 - 179 , XP009098338 ISSN: 1472-8214 abstract page 168, column 1, last paragraph - page 168, column 2, paragraph 2
- BAO ET AL: "Comparative gene expression analysis of a chronic myelogenous leukemia cell line resistant to cyclophosphamide using oligonucleotide arrays and response to tyrosine kinase inhibitors" 26 October 2007 (2007-10-26), LEUKEMIA RESEARCH, NEW YORK,NY, US, PAGE(S) 1511 - 1520 , XP022317108 ISSN: 0145-2126 page 1514, column 1, paragraph 3 page 1515, column 2, paragraph 1 page 1518, column 2, paragraph 1
- PICCALUGA P P ET AL: "Tyrosine kinase inhibitors for the treatment of Philadelphia chromosome-positive adult acute lymphoblastic leukemia" 15 September 2007 (2007-09-15), CANCER 20070915 US, VOL. 110, NR. 6, PAGE(S) 1178 - 1186 , XP002516529 ISSN: 0008-543X 1097-0142 page 1183, column 2, paragraph 3 - page 1184, column 1, paragraph 2
- KANTARJIAN HAGOP ET AL: "NILOTINIB IN IMATINIB-RESISTANT CML AND PHILADELPHIA CHROMOSOME-POSITIVE" 15 June 2006 (2006-06-15), NEW ENGLAND JOURNAL OF MEDICINE, MASSACHUSETTS MEDICAL SOCIETY, BOSTON, MA, US, PAGE(S) 2542 - 2551 , XP009072571 ISSN: 1533-4406 the whole document

## Description

The invention relates to a combination which comprises (a) a DNA damaging agent, namely chlorambucil; and (b) 4-methyl-3-[[4-(3-pyridinyl)-2-pyrimidinyl]amino]-N-[5-(4-methyl-1H-imidazol-1-yl)-3-(trifluoromethyl)phenyl] benzamide ("nilotinib"); a pharmaceutical composition comprising such a combination and optionally at least one pharmaceutically acceptable carrier, appropriate for simultaneous, separate or sequential use, in particular for use in the treatment chronic lymphocytic leukemia (CLL); and a commercial package or product comprising such a combination, each as defined in the independent claims which are incorporated here by reference.

The DNA damaging agent in a combination according to the invention is a nitrogen mustard analogue named chlorambucil, but the disclosure also allows for chlornaphazine, estramustine, mechlorethamine, mechlorethamine oxide hydrochloride, navembichin, phenestrine, prednimustine, trofosfamide, uracil mustard, cyclophosphamide, uramustine and melphalan.

US 2006/0122186 discloses a combination of imatinib with nitrogen mustard analogues and their use against CLL.

Chronic lymphocytic leukemia (CLL) is the most frequent form of leukemia in adults accounting for 25% of all leukemias (approximately 10,000 new CLL cases yearly in the United States (US)). In the US, 95% of CLL cases are B-cell phenotype leukemia. About 50% of CLL, patients are asymptomatic at diagnosis. The stage of disease correlates with prognosis; stage O having a median survival of >10 years while stage I-II has a median survival of 7 years. Treatment is usually started when patients are symptomatic.

There are two major groups of drugs utilized in the treatment of CLL: (1) alkylating agents such as chlorambucil (CLB) or cyclophosphamide and (2) purine analogs such as fludarabine. Usually chlorambucil (CLB) was the standard initial therapy, but fludarabine and cyclophosphamide (CTX) have now become the standard front-line treatment. These agents lead to responses in 60-75% of patients. Recent randomized trials demonstrated a higher response rate for fludarabine as compared to CLB but no difference in survival. Either agent is acceptable as front line therapy in CLL. Other agents are available for therapy. Eventually, all patients become resistant to the drugs. There is no therapy capable of curing this disease (Kalil, N. and Cheson, B.D. The Oncologist 4:352-369, 1999). PCT/IB 03/05454 discloses that imatinib mesylate, the active ingredient of Gleevec®, sensitizes B-CLL lymphocytes to CLB. While imatinib in combination with CLB is currently in phase I-II clinical study for the treatment of CLL, it has now surprisingly been found that nilotinib possesses a greater potency than imatinib in sensitizing CLL lymphocytes towards CLB. While not willing to be bound by the theory, we found that both nilotinib and imatinib inhibit in a similar fashion CLB-induced Rad51-related DNA repair, but only nilotinib increased CLB-induced □H2AX. Analysis of caspase-3 activation showed an increased of the apoptosis pathways mediated by JNK activation in cells treated with CLB in combination with nilotinib, but not imatinib. Moreover, c-abl inhibition by nilotinib leads to down regulation of the NFκB pathway involved in maintenance of survival B-CLL lymphocytes.

Figure: Synergistic effect of nilotinib and imatinib in CLB cytotoxicity in Lymphocytes from B-CLL patients. Evaluation of the synergistic effect of 1, 5 and 10 µM of nilotinib and imatinib in CLB cytotoxicity was assessed using MTT assay. The I value I<1 indicates that the CLB plus nilotinib or imatinib act synergistically. When I value or I>1 the drugs act antagonistically. * : p < 0.001.

The present disclosure provides a combination for simultaneous, separate or sequential use which comprises (a) DNA-damaging agent (according to the invention chlorambucil) and (b) 4-methyl-3-[[4-(3-pyridinyl)-2-pyrimidinyl]amino]-*N*-[5-(4-methyl-1H-imidazol-1-yl)-3-(trifluoromethyl)phenyl] benzamide, in which the active ingredients (a) and (b) are present in each case in free form or in the form of a pharmaceutically acceptable salt. The present disclosure or in the case of chlorambucil the present invention further provides said combination for simultaneous, separate or sequential use. Said DNA-damaging agent is a nitrogen mustard analogue.

The present disclosure provides a combination comprising (a) a nitrogen mustard analogue selected from a group consisting of chlorambucil (in the invention embodiments), chlornaphazine, estramustine, mechlorethamine, mechlorethamine oxide hydrochloride, navembichin, phenestrine, prednimustine, trofosfamide , cyclophosphamide, uramustine, melphalan,and uracil mustard and (b) 4-methyl-3-[[4-(3-pyridinyl)-2-pyrimidinyl]amino]-*N*-[5-(4-methyl-1H-imidazol-1-yl)-3-(trifluoromethyl)phenyl] benzamide, in which the active ingredients (a) and (b) are present in each case in free form or in the form of a pharmaceutically acceptable salt. The present disclosure (in the case of the invention embodiments with chlorambucil) further provides said combination for simultaneous, separate or sequential use.

The present invention provides a combination comprising (a) chlorambucil, (b) 4-methyl-3-[[4-(3-pyridinyl)-2-pyrimidinyl]amino]-*N*-[5-(4-methyl-1H-imidazol-1-yl)-3-(trifluoromethyl)phenyl] benzamide, in which the active ingredients (a) and (b) are present in each case in free form or in the form of a pharmaceutically acceptable salt. The present invention further provides said combination for simultaneous, separate or sequential use.

The present disclorue also provides a combination comprising (a) cyclophosphamide, (b) 4-methyl-3-[[4-(3-pyridinyl)-2-pyrimidinyl]amino]-*N*-[5-(4-methyl-1H-imidazol-1-yl)-3-(trifluoromethyl)phenyl] benzamide, in which the active ingredients (a) and (b) are present in each case in free form or in the form of a pharmaceutically acceptable salt. The present disclosure further provides said combination for simultaneous, separate or sequential use.

The present disclorure also provides a combination comprising (a) fludarabine and (b) 4-methyl-3-[[4-(3-pyridinyl)-2-pyrimidinyl]amino]-*N*-[5-(4-methyl-1H-imidazol-1-yl)-3-(trifluoromethyl)phenyl] benzamide, in which the active ingredients (a) and (b) are present in each case in free form or in the form of a pharmaceutically acceptable salt. The present disclosure further provides said combination for simultaneous, separate or sequential use.

The present disclosure reports that a combination comprising a nitrogen mustard analogue selected from CLB, chlornaphazine, estramustine, mechlorethamine, mechlorethamine oxide hydrochloride, navembichin, phenestrine, prednimustine, trofosfamide or uracil mustard, particularly CLB and nilotinib, can produce a therapeutic effect which is greater than that obtainable by administration of a therapeutically effective amount of either a sole nitrogen mustard analogue, in particular CLB or nilotinib alone. More specifically and in accordance with the invention, nilotinib sensitizes B-CLL lymphocytes to the treatment with CLB.

The present disclosure pertains to a combination for simultaneous, separate or sequential use, such as a combined preparation or a pharmaceutical fixed combination, which comprises (a) a nitrogen mustard analogue (in the invention embodiments chlorambucil) and (b) nilotinib in which the active ingredients (a) and (b) are present in each case in free form or in the form of a pharmaceutically acceptable salt, and optionally at least one pharmaceutically acceptable carrier.

The term "a combined preparation", as used herein defines especially a "kit of parts" in the sense that the combination partners (a) and (b) as defined above can be dosed independently of each other or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. The time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the combination partners (a) and (b), in particular in the invention embodiments a synergism, e.g. a more than additive effect, additional advantageous effects, less side effects, a combined therapeutic effect in a non-effective dosage of one or both of the combination partners (a) and (b), and very preferably a strong synergism of the combination partners (a) and (b).

The term "treatment" comprises the administration of the combination partners to a warm-blooded animal in need of such treatment with the aim to cure the disease or to effect a delay of progression of a disease.

The term "delay of progression" as used herein means that the disease progression is at least slowed down or hampered by the treatment and that patients exhibit higher survival rates than patients not being treated or being treated with the monotherapy.

The term "nitrogen mustard analogue", as commonly understood by a skilled person, refers to a cytotoxic chemotherapy agent which non-specifically alkylates DNA. Preferably the term "nitrogen mustard analogue"refers to a group of compounds, including but not limited to CLB (in the invention embodiments), chlornaphazine, estramustine, mechlorethamine, mechlorethamine oxide hydrochloride, navembichin, phenestrine, prednimustine, trofosfamide, uracil mustard cyclophosphamide, uramustine, and melphalan.

The term "chlorambucil-resistant chronic lymphocytic leukemia" as used herein defines especially a chronic lymphocytic leukemia in which CLB is no longer efficient or shows a reduction of its therapeutic effectiveness.

CLB can be prepared according to the process described in US patent 3,046,301.

4-Methyl-3-[[4-(3-pyridinyl)-2-pyrimidinyl]amino]-*N*-[5-(4-methyl-1H-imidazol-1-yl)-3-(trifluoromethyl)phenyl] benzamide is also known under the international non-proprietary name "nilotinib". It can be prepared and administered as described in WO 04/005281.

Nilotinib can be employed in the form of its mono-hydrochloride mono-hydrate as disclosed in W02007/015870.

The structure of the active agents cited may be taken from the actual edition of the standard compendium "The Merck Index" or from databases, e.g. Patents International (e.g. IMS World Publications). Any person skilled in the art is fully enable, based on these references, to manufacture and test the pharmaceutical indications and properties in standard test models, both *in vitro* and *in vivo.*

A combination as disclosed in the present invention, namely a combination which comprises (a) the nitrogen mustard analogue CLB and (b) nilotinib in which the active ingredients are present in each case in free form or in the form of a pharmaceutically acceptable salt and optionally at least one pharmaceutically acceptable carrier, will be referred to hereinafter as a COMBINATION OF THE INVENTION.

The COMBINATIONS OF THE INVENTION exhibit beneficial effects in the treatment of CLL. In one preferred embodiment of the invention, the proliferative disease to be treated with a COMBINATION OF THE INVENTION is CLL, which is resistant to CLB.

Surprisingly, the COMBINATIONS OF THE INVENTION is also better tolerated by CLL patients than the corresponding combinations employing imatinib mesylate instead of nilotinib or a pharmaceutically acceptable salt thereof.

It can be shown by established test models that a COMBINATION OF THE INVENTION results in the beneficial effects described herein before. The person skilled in the pertinent art is fully enabled to select a relevant test model to prove such beneficial effects. The pharmacological activity of a COMBINATION OF THE INVENTION may, for example, be demonstrated in a clinical study or in a test procedure as essentially described hereinafter.

Suitable clinical studies are in particular randomized, double-blind, parallel studies in CLL patients with late stage disease. Such studies are, in particular, suitable to compare the effects of a mono-therapy using the active ingredients independent of each other and a therapy using a COMBINATION OF THE INVENTION, and to prove in particular the synergism of the active ingredients of the COMBINATIONS OF THE INVENTION. The primary endpoints in such studies can be the performance status, Quality of Life scores or time to progression of the disease. In a suitable study design, patients are, for example, receiving per treatment cycle of 2 weeks, daily at a dose ranging from 50 to 1000 mg of the nilotinib and CLB at a dose ranging from 0.2 to 1 mg/kg/day.

It is one objective of this invention to provide a pharmaceutical composition comprising a quantity, which is jointly therapeutically effective against CLL comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising at least one pharmacologically active combination partner alone or in combination with one or more pharmaceutically acceptable carries, especially suitable for enteral or parenteral application. In one embodiment of the invention, one or more of the active ingredients are administered orally.

In particular, a therapeutically effective amount of each of the combination partners of the COMBINATION OF THE INVENTION may be administered simultaneously or sequentially and in any order, and the components may be administered separately or as a fixed combination. For example, a method for delaying the progression or treatment of CLL according to the present invention may comprise (i) administration of the first combination partner in free or pharmaceutically acceptable salt form and (ii) administration of the second combination partner in free or pharmaceutically acceptable salt form, simultaneously or sequentially in any order, in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the stage of CLL being treated and the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

CLB can be administered at a dose range of 0.1 to 1 mg/kg/day, preferably at a dose range of 0.2 to 0.8 mg/kg/day. Most preferably, CLB is administered at a dose of 0.6 mg/kg/day. Depending on species, age, individual condition, mode of administration, and the clinical picture in question, daily doses of about 50 to 1000 mg of nilotinib are administered to warm-blooded animals of about 70 kg bodyweight.

The disclosure relates also to a method for administering to a human subject suffering from CLL nilotinib or a pharmaceutically acceptable salt thereof.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise.

The COMBINATION OF THE INVENTION can be a combined preparation or a pharmaceutical composition.

Furthermore, the present disclosure pertains to the use of a COMBINATION OF THE INVENTION for the treatment of CLL and (according to the invention) for the preparation of a medicament for the treatment of CLL.

Additionally, the present invention pertains to the use of CLB in combination with nilotinib for the preparation of a medicament for the treatment of CLL.

Moreover, the present invention provides a commercial package comprising as active ingredients COMBINATION OF THE INVENTION, together with instructions for simultaneous, separate or sequential use thereof in the treatment of CLL.

### Example I: Nilotinib sensitizes CLL lymphocytes to CLB

### A-Material and Methods

**A-1) Isolation of CLL** Lymphocytes and cell culture Lymphocytes are isolated from the peripheral blood of CLL patients by sedimentation centrifugation on Ficoll Hypaque (Pharmacia, Uppsala, Sweden) as described previously (Christodoulopolis G. et al., Cancer Research, 1999, 5:2178-84). Aliquots containing 1x10⁶ cells/ml are sent for T-lymphocyte analysis. The percentage of contaminating T lymphocytes is determined using fluorescence-activated cell sorting analysis with CD3 antibody. The percentage of T-lymphocyte contamination in our population (expressed as a mean% ±SE) is 6.4±1.8.
   The WSU cell line is a B-lymphocytic cell line derived from a CLL patient (Mohammad R.M., et al., Leukemia, 1996, 10:130-7).
**A-2) Plating Efficiency and Dosing.** The lymphocytes and WSU CLL lymphocytes are seeded into 96-well plates in 200 µl suspensions containing 1.5x10⁶ lymphocytes/ml and 1.25x10⁵ cells/ml respectively in RPMI supplemented with 10% FBS. Only dose responses with linear plating efficiencies are analyzed. The lymphocytes are then incubated at 37° C in the presence of various concentrations of nilotinib (0-100 µM) alone, CLB (0-100 µM) alone, or various concentrations of both drugs together.
**A-3) Cytotoxic Assay.** The MTT assay is performed 72 hours after plating as described before (Christodoulopolis G et al., Cancer Research, 1998, 58:1789-92) by addition of 20 µl of a solution of 5 mg/ml MTT (3-[4,5-dimethylthiazol-2-yl]2,5-diphenyltetrazolium bromide) in RPMI media to each well. The LD₅₀ of CLB alone, nilotinib alone or CLB in the presence of nilotinib is defined as to be the drug concentration required to reduce the absorbance reading to 50% of the control value. The percentage of surviving cells after treatment respect to vehicle treated cells (control) is calculated as (OD treated cells/OD untreated cells)x100. Synergy is determined by the formula: a/A + b/B = 1 where a is the concentration of CLB required to produce 50% of control values in combination with nilotinib at concentration b; A is the concentration of CLB that produces an LD₅₀ without nilotinib; and B is the concentration of nilotinib that produces an LD₅₀ in the absence of CLB. According to the formula, when I<1 the interaction is synergistic, when I = 1, the interaction is additive, and when I>1 there is an antagonistic interaction.
A-4) Statistical Analysis Differences between mean values is assessed by two tailed t-test. Correlation and linear regression analysis are performed using the EXCEL Statistical Tool Package.

### Results

Nilotinib possessed greater potency than imatinib in sensitizing CLL lymphocytes (16/19 samples) towards chlorambucil.

### Example II

Patients. Twenty one patients with a diagnosis of B-CLL followed at the Jewish General Hospital of Montreal were enrolled in the study after informed consent. Patients were either clinically untreated (n=13) or treated with CLB (n=8) for various time periods.

**Cytotoxicity assay.** Lymphocytes were isolated from the peripheral blood using Ficoll-Hypaque (Pharmacia). The T-lymphocyte contamination in the isolated B-lymphocytes population was 2.30±2.07 (expressed as a mean % ± S.D. and determined by flow cytometry analysis). The CLL lymphocytes (3x10⁶ cells/ml) were plated in RPMI 1640 supplemented with 10% FBS and incubated in the presence of various concentrations (0-100 µM) of imatinib (Novartis), nilotinib (Novartis), chlorambucil alone (Sigma-Aldrich Co), or in combination as indicated. Control samples were incubated with the greatest volume of DMSO. The MTT assay was performed 72 h after treatment as previously described ( Christodoulopoulos et al, Cancer Res, 58: 1789-1792, 199835). Synergy was determined by the formula: a/A + b/B = I, where a is the CLB IC₅₀ (concentration resulting in 50% of control) in combination with imatinib or nilotinib at concentration b; A is the CLB IC₅₀ without imatinib or nilotinib; and B is the imatinib or nilotinib IC₅₀ in the absence of CLB. According to the formula, when I<1, the interaction is synergistic, when I=1, the interaction is additive, and when I>1 there is an antagonistic interactio.

**Statistical analysis.** Statistical analysis by ANOVA and *t-test* were done with SigmaStat software (Systat Software Inc., San Jose, CA, USA).

### Results

### Both c-abl inhibitors sensitized B-CLL lymphocytes to chlorambucil

A total of 21 CLL patients were enrolled on this study. Thirteen patients were clinically untreated (patients 1 to 13) and eight had received prior therapy with chlorambucil (treated patients 14 to 21). Their median age was 72 years (range 45 to 90 years) and their median WBC count was 83.7x10⁹ cells/liter (range 32.87x10⁹ to 256.27x10⁹ cells/liter).

The MTT assay was utilized to determine the cytotoxicity of CLB alone, imatinib alone, nilotinib alone or the combination of CLB with 1, 5 or 10 µM imatinib or nilotinib in lymphocytes from CLL patients.

When used at 1, 5 or 10 µM, nilotinib sensitized (synergistic plus additive effect) CLL lymphocytes to CLB in 84.6%, 100% and 90.5% of the patients tested respectively, while imatinib sensitized only 66.7%, 68.4% and 36.8% of these samples to CLB at similar concentrations. Interestingly, sensitization of B-CLL lymphocytes to CLB is statistically more potent with 5 or 10 µM nilotinib than with imatinib **(figure).**

Furthermore, Five out of the twenty one patients were selected in their ability to sensitize B-CLL lymphocytes to CLB. Using the MTT assay, we evaluated the effect of nilotinib and imatinib on CLB cytotoxicity in malignant B lymphocytes from these five patients. The I-value, I<1 or I>1, indicates that the CLB and c-abl inhibitors act synergistically or antagonistically, respectively.

**Table:**

| **Patient** | **CLB IC₅₀ (µM)** | **Nilotinib IC₅₀ (µM)** | **Imatinib IC₅₀ (µM)** | **CLB+5µM nilotinib IC₅₀ (µM)** | **Synergy Value I** | **CLB+5µM imatinib IC₅₀ (µM)** | **Synergy Value I** |
|---|---|---|---|---|---|---|---|
| 9 | 11.68 | >100 | 43.32 | 8.19 | 0.70 | 12.43 | 1.18 |
| 14 | 8.10 | 33.16 | 42.07 | <2.5 | 0.38 | 6,07 | 0.87 |
| 15 | 49.69 | 19.92 | 37.92 | 41.06 | 1.08 | 66.60 | 1.47 |
| 16 | 4.54 | 9.61 | 14.22 | <2,5 | 0.68 | 3.51 | 1.12 |
| 18 | 66.02 | 28.73 | 38.35 | 37.13 | 0.69 | 55.56 | 1.00 |

| | | | | | | | |
|---|---|---|---|---|---|---|---|
| Conclusion: We now demonstrate that nilotinib, a more potent c-abl inhibitor than imatinib, has a greater efficacy to synergize CLB cytotoxicity in B-CLL lymphocytes. | | | | | | | |

## Claims

1. A combination for simultaneous, separate or sequential use which comprises (a) DNA-damaging agent and (b) 4-methyl-3-[[4-(3-pyridinyl)-2-pyrimidinyl]amino]-N-[5-(4-methyl-1 H-imidazol-1-yl)-3-(trifluoromethyl)phenyl] benzamide, in which the active ingredients (a) and (b) are present in each case in free form or in the form of a pharmaceutically acceptable salt, and wherein the DNA-damaging agent is chlorambucil.

2. The combination according to any one of the preceding claims, wherein compound (b) is used in the form of its mono-hydrochloride mono-hydrate.

3. A combination according to any one of the claims 1 or 2 for use in a method of treatment of chronic lymphocytic leukemia.

4. The combination according to claim 3 wherein the use is in a method of treatment of chronic lymphocytic leukemia that is resistant to chlorambucil.

5. A pharmaceutical composition comprising a combination according to any one of the claims 1-4 and at least one pharmaceutically acceptable carrier.

6. A commercial package comprising a pharmaceutical composition according to claim 5 together with instructions for simultaneous, separate or sequential use thereof in the treatment of chronic lymphocytic leukemia.

## Patentansprüche

1. Kombination zur gleichzeitigen, separaten oder sequentiellen Verwendung, die (a) ein DNAschädigendes Mittel und (b) 4-Methyl-3-[[4-(3-pyridinyl)-2-pyrimidinyl]amino]-*N*-[5-(4-methyl-1H-imidazol-1-yl)-3-(trifluormethyl)phenyl]benzamid umfasst, wobei die Wirkstoffe (a) und (b) jeweils in freier Form oder in Form eines pharmazeutisch akzeptablen Salzes vorliegen, und wobei das DNAschädigende Mittel Chlorambucil ist.

2. Die Kombination nach einem der vorhergehenden Ansprüche, wobei die Verbindung (b) in Form ihres Monohydrochloridmonohydrats verwendet wird.

3. Kombination nach einem der Ansprüche 1 oder 2 zur Verwendung in einem Verfahren zur Behandlung chronischer lymphatischer Leukämie.

4. Die Kombination nach Anspruch 3, wobei die Verwendung in einem Verfahren zur Behandlung chronischer lymphatischer Leukämie besteht, die chlorambucilresistent ist.

5. Pharmazeutische Zusammensetzung, die eine Kombination nach einem der Ansprüche 1 bis 4 und zumindest einen pharmazeutisch akzeptablen Träger umfasst.

6. Kommerzielle Packung, die eine pharmazeutische Zusammensetzung nach Anspruch 5 samt Anweisungen zur gleichzeitigen, separaten oder sequentiellen Verwendung davon bei der Behandlung chronischer lymphatischer Leukämie umfasst.

## Revendications

1. Combinaison pour une utilisation simultanée, séparée ou séquentielle qui comprend (a) un agent d'endommagement d'ADN et (b) du 4-méthyl-3-[[4-(3-pyridinyl)-2-pyrimidinyl]amino]-N-[5-(4-méthyl-1H-imidazol-1-yl)-3-(trifluorométhyl)phényl]benzamide, dans laquelle les principes actifs (a) et (b) sont présents dans chaque cas sous forme libre ou sous la forme d'un sel pharmaceutiquement acceptable, et dans laquelle l'agent d'endommagement d'ADN est le chlorambucil.

2. Combinaison selon l'une quelconque des revendications précédentes, dans laquelle le composé (b) est utilisé sous la forme de son mono-chlorhydrate mono-hydrate.

3. Combinaison selon l'une quelconque des revendications 1 ou 2, pour l'utilisation dans une méthode de traitement de la leucémie lymphoïde chronique.

4. Combinaison selon la revendication 3, dans laquelle l'utilisation est dans une méthode de traitement de la leucémie lymphoïde chronique qui est résistante au chlorambucil.

5. Composition pharmaceutique comprenant une combinaison selon l'une quelconque des revendications 1 à 4 et au moins un support pharmaceutiquement acceptable.

6. Emballage commercial comprenant une composition pharmaceutique selon la revendication 5 conjointement avec des instructions pour une utilisation simultanée, séparée ou séquentielle de celle-ci dans le traitement de la leucémie lymphoïde chronique.
